# EUROPEAN PATENT SPECIFICATION

(11) **EP 0 915 874 B1**
(45) Date of publication and mention of the grant of the patent: **10.12.2003**
(21) Application number: 97937496.4
(22) Date of filing: 17.07.1997
(51) Int. Cl.: C07D 473/00, A61K 31/52

(54) **PROCESS OF PREPARATION OF VALACYCLOVIR AND RELEVANT INTERMEDIATES**
VERFAHREN ZUR HERSTELLUNG VON VALACYCLOVIR UND RELEVANTE ZWISCHENVERBINDUNGEN
PROCEDE DE PREPARATION DE VALACYCLOVIR ET DE PRODUITS INTERMEDIAIRES PRESENTANT UN INTERET PARTICULIER

(30) Priority: 18.07.1996 IT MI961491
(43) Date of publication of application: 19.05.1999
(73) Proprietor: INDUSTRIALE CHIMICA S.r.l., 20145 Milano (IT)
(72) Inventor: MONTORO, Maurizio, I-20014 Nerviano (IT); PIROVANO, Silvio, I-21013 Gallarate (IT)
(74) Representative: Gervasi, Gemma, Dr.
(86) International application number: EP9703826
(87) International publication number: WO98003553

(56) References cited:
- EP-A- 0 308 065
- WO-A-94/29311
- WO-A-96/22291

## Description

### Field of the invention

The present invention relates to a process of preparation of valacyclovir and relevant intermediates.

### State of the art

Valacyclovir, i.e. 2-[(2-amino, 1,6-dihydro, 6-oxo, 9-H, purin-9-yl)methoxy]ethyl, L-valinate of formula (I) 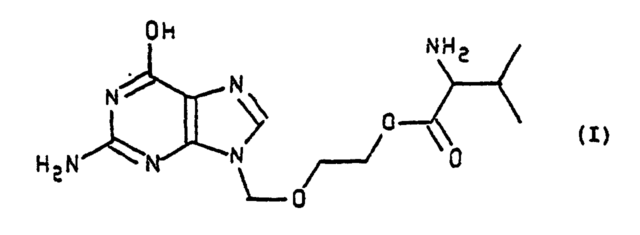 is the ester of acyclovir, i.e. of 2-amino, 1,6-dihydro-6-oxo, 9-H, 9[(2-hydroxyethoxy)methyl]purine of formula (II) with L-valine.

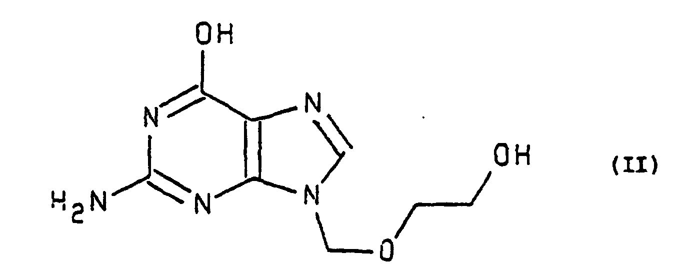

Said substance, which is hydrolyzed to acyclovir by the body, may be administered by the oral way.

Several esters of acyclovir with amino acids are reported to be effective "prodrugs" of acyclovir; among them, the ester with L-valine is the most absorbable and, therefore, provides high acyclovir concentrations in the body.

The use of valacyclovir produces acyclovir concentrations in the plasma equivalent to those obtained by i.v. administration, the antiviral activity of acyclovir and its efficacy over a broader spectrum of herpetic infections being thus increased.

European Patent 0 308065 B1 describes valacyclovir and in particular the two processes of preparation of said active ingredient reported below:
1) condensation of acyclovir with N-benzyloxycarbonyl valine in the presence of a condensing agent, such as dicyclohexylcarbodiimide, and successive removal of the protecting group by hydrogenolysis. i.e. according to the following scheme (1) 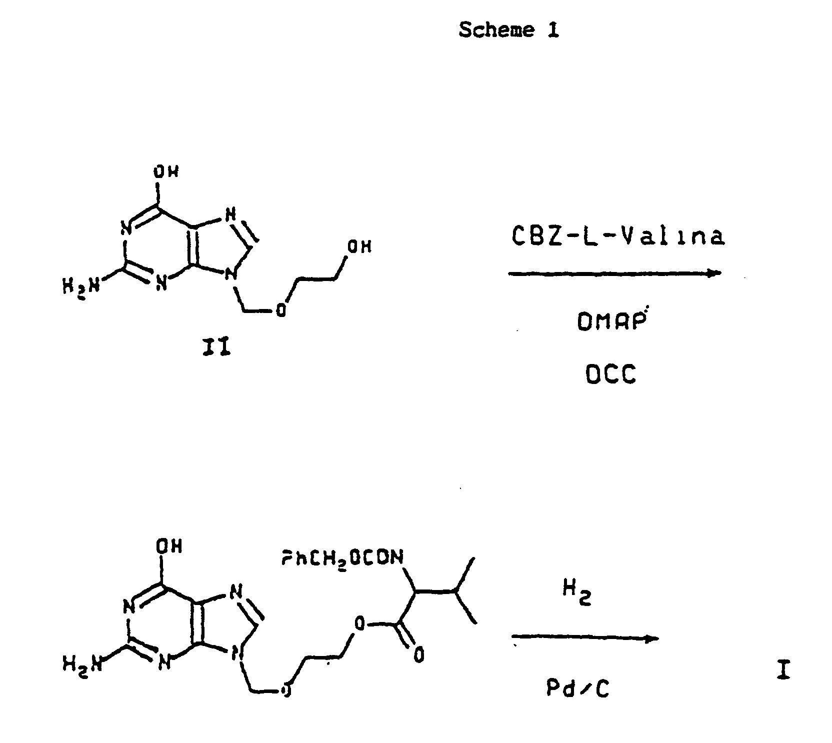
2) condensation of guanine (III), in which X is a protecting group of the hydroxyl function, Q is H, an acetyl, benzoyl or trimethylsilyl group, with the product of formula ACH₂OCH₂CH₂OCH(NHR¹)CH(CH₃)₂, in which A is a leaving group, such as a halogen atom (Br or Cl), acetate or benzoate, R¹ is a protecting group of the aminic function, i.e. according to the following scheme (2)

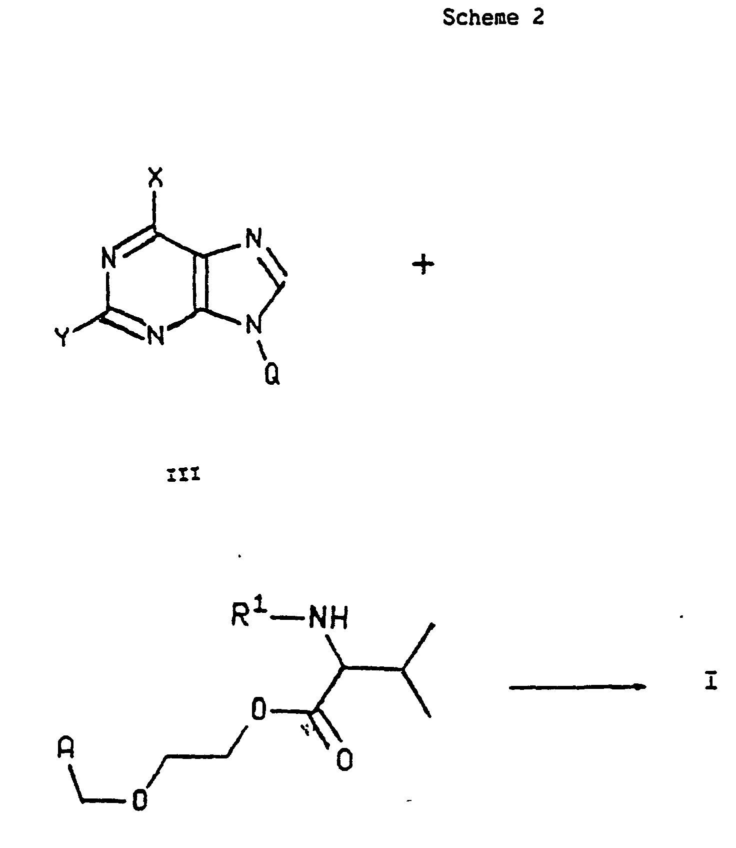

There are several drawbacks to the aforesaid processes: actually they require very costly reactants, such as benzyloxy carbonyl chloride, dicyclohexylcarbodiimide, palladium for hydrogenation, as well as specific plants, such as a pressure hydrogenator, which conditions prevent the aforesaid processes from being easily sealed up to industrial size.

PCT Application WO-94/2931 relates to a process for the preparation of an amino acid ester of a nucleoside analogue having an esterifiable hydroxy group in its linear or cyclic ether moiety, said process comprising the reaction of the nucleoside analogue with a 2-oxa-4-aza-cycloalkane-1,3-diane of formula 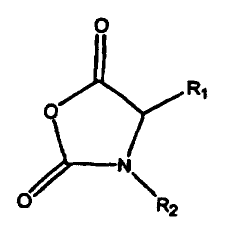 wherein R₁ is H, C₁₋₄-alkyl or alkenyl group or other amino acid side chains, R₂ is H or -COOR₃, where R₃ is a benzyl, t-butyl, fluorenylmethyl or an optionally halo-substituted linear or branched C₁₋₈-alkyl group.

Therefore, the need for a process of synthesis allowing the obtainment of high-purity valacyclovir in high yields, free from the drawbacks of the processes known in the prior art and easily scaled up to commercial size was deeply felt.

### Summary

It has surprisingly been found a process of preparation of high-purity valacyclovir of formula (I) or of a salt thereof of formula (I-A), in high yields, 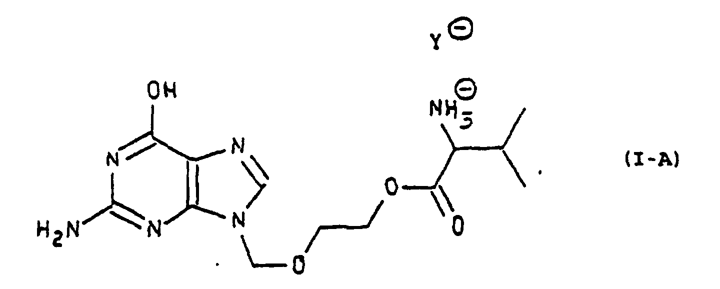 in which Y^{⊖} is selected from the group consisting of Cl^{⊖}, HSO₄^{⊖}, paratoluenesulfonate, methanesulfonate, trifluoromethane-sulfonate, free from the drawbacks of the processes known and, therefore, easily scalable to industrial dimensions.

In particular, the process of the present invention comprises the following steps:
a) reacting acyclovir (II) with a Z-L reactant selected from the group consisting of para-toluenesulfonyl chloride, methanesulfonyl chloride, trifluoromethanesulfonyl chloride, triflic anhydride, sulfuryl chloride, phosphorus tribromide and trichloride, in the presence of a base neutralizing the acidity that forms during the reaction, thereby obtaining a compound of formula (IV) 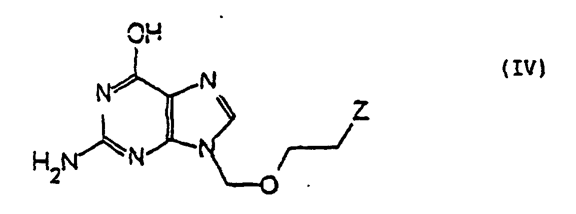 in which Z is selected from the group consisting of paratoluenesulfuryl (0-Tosyl), methanesulfuryl (0-mesyl), trifluoromethanesulfuryl 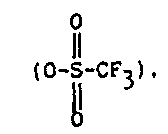 Cl,Br;
b) reacting product (IV) coming from the preceding step with an alkaline salt of valine of formula (V) 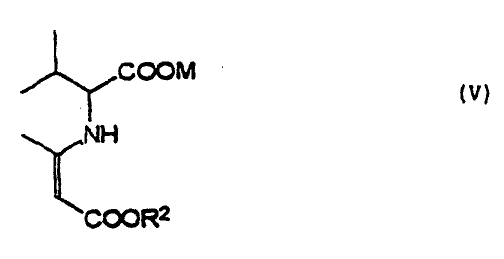 in which R² is a C₁-C₁₀ alkyl and M is an alkali metal,
   in the presence of a dipolar aprotic high-boiling solvent or a high-boiling ether, at a temperature ranging from 20°C to 150°C, to give ester (VI) 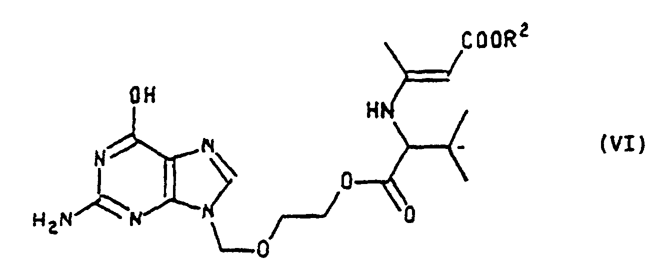 in which R² has the above meanings;
c) transforming product (VI) coming from the preceding step into the corresponding valacyclovir hydrochloride (I-A) by acid hydrolysis with a strong acid of mineral or organic type, at a temperature ranging from 0°C to 50°C, in water and/or in an organic solvent selected from the group consisting of the solvent used in step (b), an alcohol containing 1 to 4 carbon atoms, an ether, and a mixture of said alcohol and said ether;
d) optionally converting valacyclovir hydrochloride (I-A) into the corresponding free base (I) by conventional methods.

In particular, it has surprisingly been found that, in step (a) of the process according to the present invention, product (IV) is selectively formed in high yields, whereas there is no formation of by-products deriving from the possible reaction of the Z-L reactant with the aminic group at the 2 position and/or with the hydroxyl at the 6 position of the purinic ring.

Also, this invention extends to intermediates (IV) and (VI) obtained by the process according to the present invention.

The present invention also relates to an alkaline salt of L-valine (V), in which M has the above meanings, used as a reactant in step (b) of the process of the present invention, and the relevant process of synthesis, which, in particular, includes the reaction of L-valine in the presence of an alkaline hydroxide with an alkyl acetatoacetate of formula (VII) 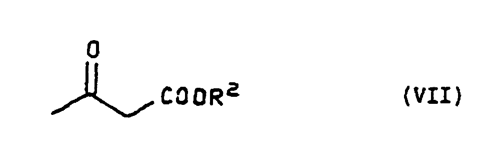 in which R² has the above meanings, in a solvent selected from among an alcohol containing 1 to 4 carbon atoms, a ketone or an aromatic hydrocarbon.

### Description of the figures

Fig. 1 shows the I.R. spectrum, run KBr, containing 1% of hydrated valacyclovir hydrochloride (I-A) prepared as described in Examples 6 and 9.
Fig. 2 shows the I.R. spectrum, run KBr, containing 1% of anhydrous valacyclovir hydrochloride (I-A) prepared as described in Example 11.

### Detailed description of the invention

The base used in step (a) of the process according to the invention is preferably selected from potassium carbonate, sodium methylate and pyridine.

The solvent used in step (a) is preferably N,N-dimethylformamide or dimethylsulfoxide or pyridine.

According to particularly preferred embodiments, pyridine or a pyridine-dimethylformamide mixture is used in step (a) as a solvent or as a base.

According to a particularly preferred embodiment, para-toluenesulfonyl chloride or methanesulfonyl chloride is used in step (a) as a Z-L reactant.

Should para-toluenesulfonyl chloride be used as a Z-L reactant, the process of the present invention will proceed according to Scheme 3

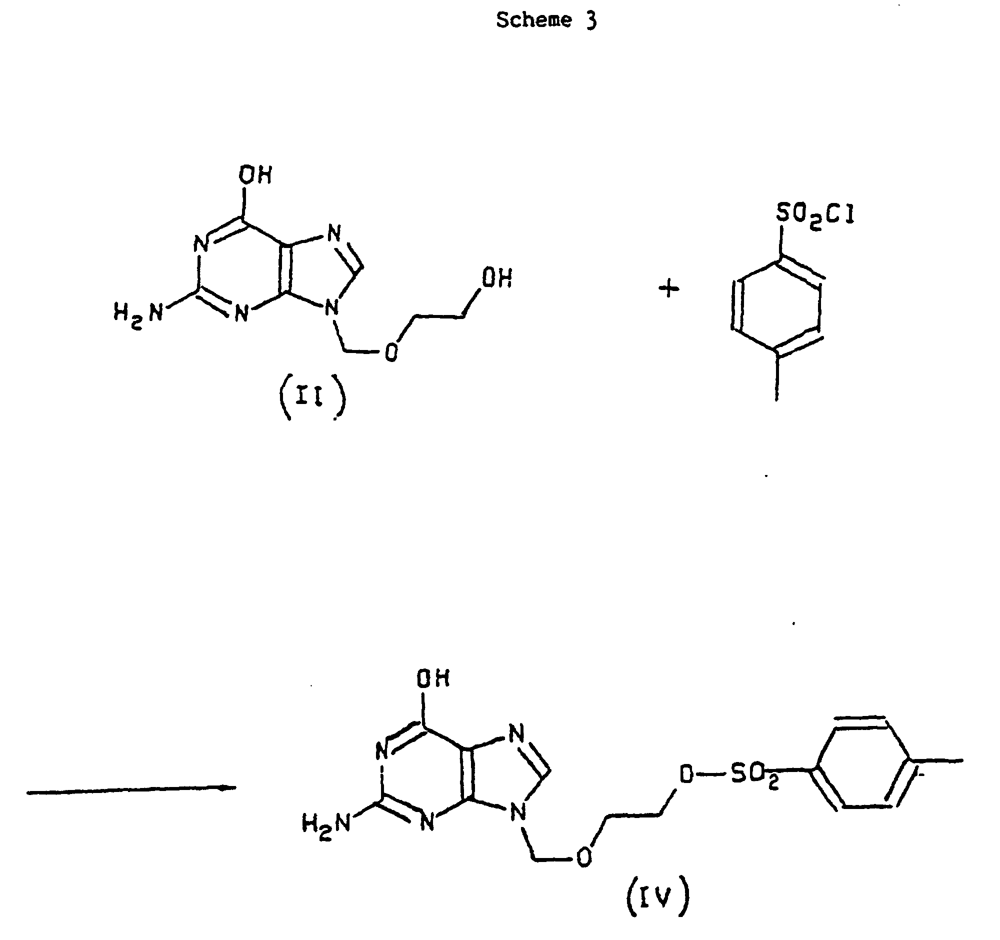

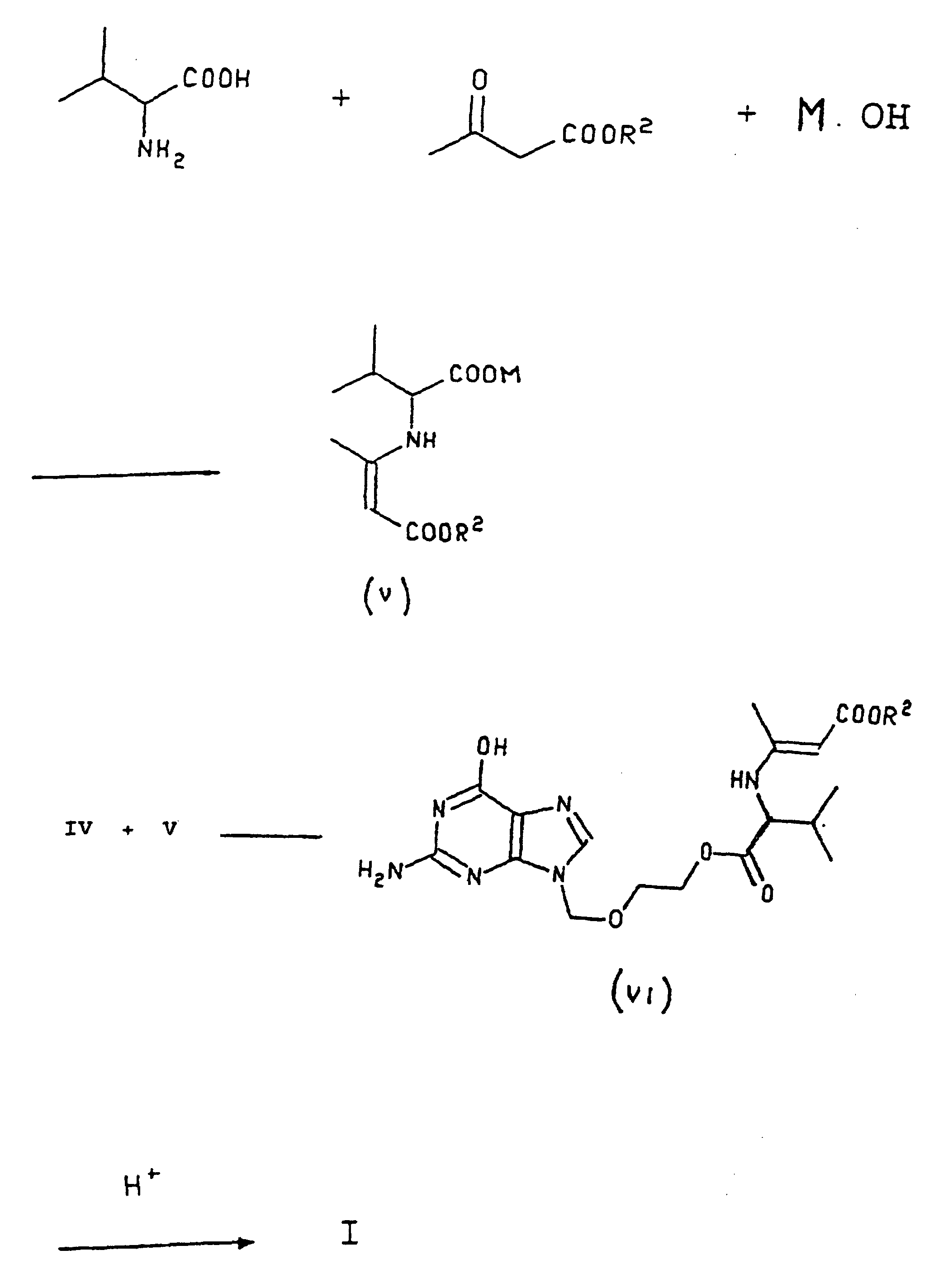

In step (a), omega-chloroacyclovir may be obtained not only by reacting acyclovir with PCl₃, but also by treating omega-tosyl or mesyl acyclovir in the presence of LiCl.

When a dipolar aprotic solvent is used in step (b) according to the present invention, said solvent is preferably N.N-dimethylformamide or dimethylsulfoxide.

When an ether high-boiling i.e. boiling at temperatures ≥ 100°C is used in step (b), said ether is preferably dioxane or diglyme.

Step (b) is preferably carried out at a temperature ranging from 70°C to 90°C.

In step (b), when the reactant used is intermediate (IV), in which Z=Cl (omega-chloroacyclovir), a further reactant, i.e. potassium iodide, is added.

Product (VI), recovered from the reaction mixture, can be used in successive step (c) without purification.

When a mineral strong acid is used in step (c) of the claimed process, said acid is preferably sulfuric acid or hydrochloric acid. When, in said step, an organic strong acid is used, this is preferably para-toluenesulfonic acid or methanesulfonic acid or trifluoromethanesulfonic acid. In this step, the acid may be added either in stoichiometric or catalytic amounts.

According to a particularly preferred embodiment, the aforesaid acid is added in quantities securing constant pH values of 2 to 3.

When, in step (c), an alcohol containing 1 to 4 carbon atoms is used as a solvent, said alcohol is preferably methanol. Instead, when an ether is used, said ether is preferably dioxane or tetrahydrofuran.

When a mixture of the aforesaid organic solvents is used, said mixture preferably consists of methanol and tetrahydrofuran.

According to a particularly preferred embodiment, step (c) is directly conducted on the reaction mixture obtained in (b), i.e. in the presence of the same solvent as used in (b), without isolating intermediate (VI). Valacyclovir may be used as an active ingredient in the form of a salt (I-A), preferably the hydrochloride salt, or may be transformed, according to conventional techniques, into the corresponding free base (I), as envisaged in step (d) of the present invention.

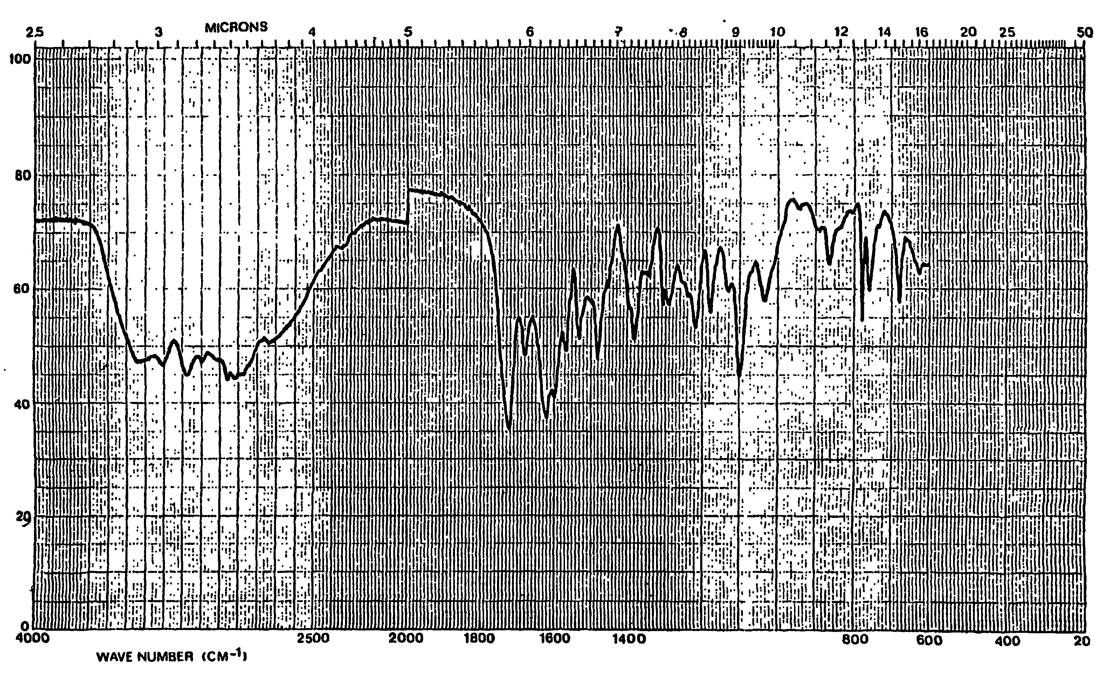

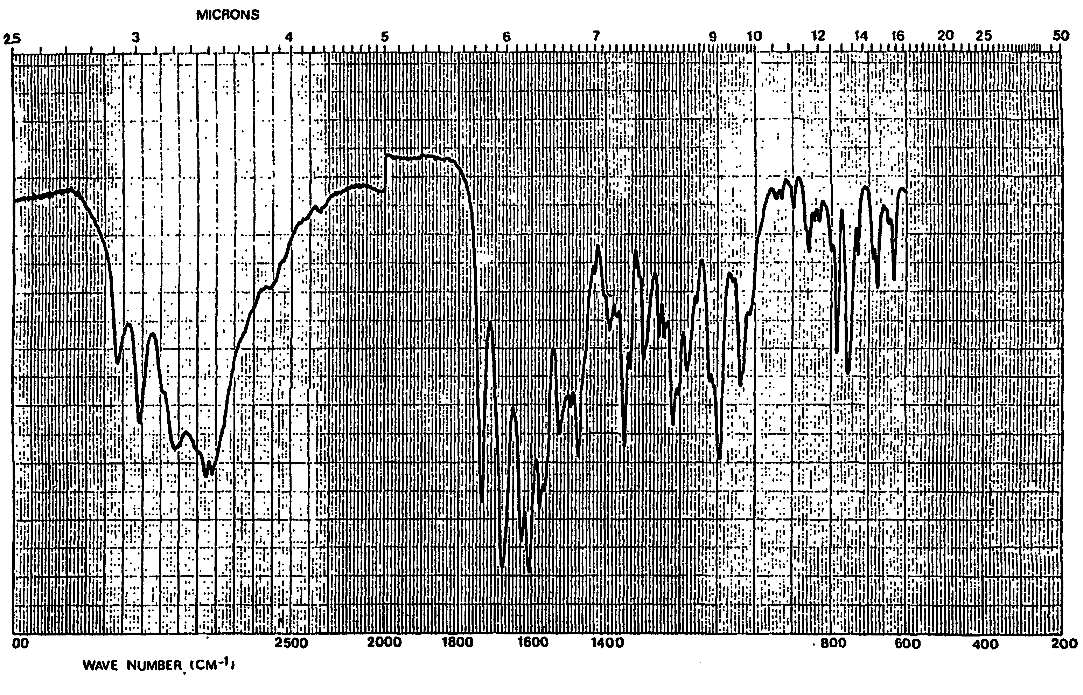

In particular, valacyclovir hydrochloride exists in two crystalline modifications identified by different I.R. spectra (Fig. 1 and, respectively, Fig. 2).

A crystalline modification may have a water content that varies from 2% to 10% with the atmospheric moisture content. The other has a low water content (lower than 2%), which does not significantly change by exposure to atmospheric moisture.

Either one of the two modifications is obtained depending on the operating conditions, in particular on the water content of the crystallization solvents.

According to a particularly preferred embodiment, step (d) includes the addition of an ammonium hydroxide aqueous solution to a valacyclovir hydrochloride solution (I-A) to adjust the pH to a constant value ranging from 9 to 9.5.

Particularly preferred alkaline salts of L-valine (V) according to the present invention are those in which M is sodium and/or potassium and R² is methyl, ethyl, n-propyl, isopropyl, n-butyl, isobutyl.

Should their formation take place in a short-chain alcohol, this will preferably be methanol; should it take place in a ketone, this will preferably be methylethylketone or diethylketone or methylisobutylketone; should it take place in an aromatic hydrocarbon. this will preferably be toluene or xylene.

In the process of preparation of the alkaline salt according to the present invention, an alkyl acetoacetate (VII), in which R² is preferably methyl, ethyl, n-propyl, isopropyl, n-butyl, isobutyl, is used.

The following examples only illustrate the process of preparation of valacyclovir according to the invention as well as the process of preparation of the corresponding L-valine alkaline salt (V).

### Example 1 - L-valine sodium salt (V)

A 500-ml four-neck flask, equipped with mechanical stirrer, thermometer, reflux with calcium chloride valve and inlet for nitrogen, was fed, at room temperature and under a nitrogen stream, with 110 ml methylethylketone and 10 g L-valine.

The resulting suspension was added with 10.1 ml methyl acetoacetate and 3.52 g NaOH (99%) under stirring at room temperature and under a nitrogen stream. No solution was obtained.

The reaction mixture was heated to reflux (78°C-80°C) for 3 h (a complete solution was obtained already at 50°C), and then concentrated in a rotating evaporator by means of water vacuum. Solvent traces were first removed by toluene addition to the residue followed by solvent distillation under vacuum and then by treatment of the residue with ethyl ether followed by solvent distillation in a rotating evaporator under water vacuum.

The residue obtained was treated with 200 ml ethyl ether, filtered through a Buchner funnel and washed with fresh ethyl ether.

The hygroscopic solid obtained was mashed and recrystallized from hexane (200 ml) at room temperature.
18.7 g of product was obtained (yield 92.5%).
Perchloric acid grade: 93.9%.
Water content (KF): 5.6%.

The ¹H NMR spectrum of the compound showed signals at δ 0.85 (6H,t); 1.80 (3H,s); 2.00 (1H,m); 3.45 (3H,s); 3.50 (1H,m); 4.20 (1H,s); 8.85 (1H,d).

### Example 2 - L-valine potassium salt (V)

A 500-ml four-neck flask, equipped with mechanical stirrer, thermometer, reflux with calcium chloride valve and dropping funnel, was fed at room temperature with 240 ml methanol and 10 g L-valine. No solution was obtained.

5.1 g KOH (92%) dissolved in 100 ml methanol were added dropwise at room temperature, under a nitrogen stream. The mixture was heated to boiling. A complete solution was obtained, kept at that temperature for 45 min and then allowed to cool to 25°C-30°C. At that temperature and under a nitrogen stream, 10.3 ml methyl acetoacetate dissolved in 20 ml methanol were added dropwise. The resulting mixture was heated to reflux and kept at that temperature for 2 h. Methanol was removed under water vacuum to give a doughy residue, which was dissolved with acetone until formation of a crystalline solid, which was filtered through a Buchner funnel and 25 oven dried at 50°C.
16.20 of a hygroscopic solid was obtained (yield 75%).

### Example 3 - L-valine sodium salt (V)

A 100-ml flask, equipped with mechanical stirrer and reflux, was fed at room temperature with 3 g L-valine, 25 ml toluene and 2.05 g aqueous NaOH at 50%. The mixture was stirred for 30 min at 50°C and then added with 3.1 g methyl acetoacetate. The reaction mixture obtained was stirred for 30 min at 50°C, heated to reflux and kept at that temperature for 15 min. The product was recovered after removing the reaction solvents in a rotating evaporator under water vacuum three times, each time adding 50 ml fresh toluene, 6.3 g of product was obtained (yield 99%).

### Example 4 - omega-Tosyl acyclovir (IV)

A 250-ml four-neck flask, equipped with mechanical stirrer, thermometer, reflux with calcium chloride valve and inlet for nitrogen, was fed at room temperature with 10 g acyclovir in 60 ml pyridine. No solution was obtained.

At that temperature and under a nitrogen stream, the flask was fed with 15 g para-toluenesulfonyl chloride. The mixture was stirred at room temperature and the product slowly passed into solution. The reaction was exothermic and the highest temperature reached was 55°C; thus the reaction mixture spontaneously heated up to 50°C-55°C until reaction completion. (Overall reaction time: approx. 90 min).

The reaction was followed by TLC. After the reaction was complete, the mixture was cooled to 20°C and poured into 300 ml iced water. The pale yellow precipitated solid was filtered and washed with water.

The solid obtained was suspended in water (pH 7.2) and brought to pH 5.0 by addition of 15% HCl, while the temperature was kept at 0°C-5°C. The suspension was filtered through a Buchner funnel and the precipitate obtained was washed with water and oven dried at 45°C-50°C for 3 h.
14.8 g of a pale yellow solid was obtained (yield 87.9%).

The mass spectrum of the compound showed peaks at m/z: 379 (molecular ion), 207 (para-toluenesulfonic acid loss), 164 (-O-CH₂CH₂-OTs fragment loss), 155 (CH₃-Ph-SO₂ fragment), 150 (CH₂-O-CH₂CH₂-OTs loss).

The ¹H NMR spectrum of the compound showed signals at δ 2.4 (3H,s); 3.6 (2H,m); 4.1 (2H,m); 5.28 (2H,s); 6.5 (2H,bs) (disappears by deuteration); 7.43 (2H,m); 7.72 (2H,m); 7.76 (1H,s).

### Example 5 - Ester (VI)

A 500-ml four-neck flask, equipped with mechanical stirrer, thermometer, reflux with calcium chloride valve and inlet for nitrogen, was fed at room temperature with 10 g omega-tosyl acyclovir prepared as described in Example 4, 180 ml DMF, 7.5 g L-valine sodium salt (V) prepared as described in Example 1. The mixture was heated to 80°C for 3 h. The reaction was followed by TLC.

After the reaction was complete, the reaction mixture was concentrated in a rotating evaporator by means of an oil pump until obtaining a volume residue of 30 ml (rotating evaporator bath temperature of 70°C).

The mixture was added dropwise to 300 ml iced water, while the temperature was kept at 0°C-5°C, filtered through a Buchner funnel, washed 3 times with 50 ml water, and oven dried at 50°C. 8.5 g of product was obtained (yield 80%).

The mass spectrum of the compound showed signals at m/z: 422 (molecular ion). 308 (-NH-C(CH₃)=CH-COOCH₃) fragment loss), 164 (9-methylene guanine fragment), 151 (guanine fragment).

The ¹H NMR spectrum of the compound showed signals at δ 0.85 (6H,m); 1.82 (3H,s); 2.02 (1H,m); 3.3 (3H,s); 3.68 (2H,m); 4.06 (1H,m); 4.16 (1H,m); 4.24 (1H,m); 5.32 (2H,s); 6.3 (2H,bs) (disappears by deuteration); 7.8 (1H,s); 8.82 (1H,d).

### Example 6 - Hydrated valacyclovir hydrochloride (I-A)

A 250 ml conical flask equipped with magnetic stirrer was fed at 20°C with 4 g ester (VI) prepared as described in Example 5, in 40 ml methanol and 40 ml tetrahydrofuran. The mixture was added under stirring with 15% HCl until the solution pH was 2.0, kept at 20°C for approx. 3 h, while the pH was adjusted to a constant value ranging from 2.0 to 2.5 by addition of 15% HCl. The reaction was followed by TLC. After the reaction was complete, the reaction mixture was concentrated in a rotating evaporator under water vacuum and the residue was dissolved in 75 ml ethanol. The mixture was heated under stirring to 50°C for 30 min, allowed to cool to 0°C for 1 h, filtered, and washed with ethanol. The wet product was dried under vacuum. 2.8 g of product (I-A) having a water content of 5% was obtained (reaction yield 78% of theoretical value).

The I.R. spectrum of the product is shown in Fig. 1.

The mass spectrum of the compound showed peaks at m/z: 324 (molecular ion of the base), 281 (CH(CH₃)₂ fragment loss), 209 (-OCOCH-(NH₂)CH(CH₃)₂ fragment loss), 164 (9-methylene guanine), 151 (guanine fragment).

The ¹H NMR spectrum of the compound showed signals at δ 0.86 (6H,m); 2.07 (1H,m); 3.7 (2H,m); 4.18 (1H,m); 4.36 (1H,m); 5.36 (2H,s); 6.66 (2H,bs) (disappears by deuteration); 7.8 (1H,s).

### Example 7 - Valacyclovir base (I)

A solution of 0.5 g valacyclovir hydrochloride, prepared as described in Example 6, in 25 ml distilled water was added with 12.5% ammonium hydroxide until the solution pH was brought to a constant value of 9.0-9.5.

The resulting mixture was concentrated under vacuum at 50°C to a volume of 10 ml. After cooling, the precipitate was filtered and dried.

273 mg of a white product having the same analytical characteristics as those of a sample obtained according to European Patent 0308 056 B1 was obtained.

The mass spectrum of the compound showed peaks at m/z: 324 (molecular ion), 281 (CH(CH₃)₂ fragment loss), 209 (-OCO-CH(NH₂)CH(CH₃)₂ fragment loss), 164 (9-methylene guanine fragment), 151 (guanine fragment).

The ¹H NMR spectrum of the compound showed signals at δ 0.74 (3H,d); 0.80 (3H,d); 1.72 (1H,m); 3.05 (1H,d); 3.66 (2H,m); 4.08 (1H,m); 4.15 (1H,m); 5.32 (2H,s); 6.50 (2H,bs) (disappears by deuteration); 7.8 (1H,s).

### Example 8 - omega-mesyl acyclovir (IV)

A 250 ml flask was fed with 30 ml pyridine, 30 ml DMF and 10 g acyclovir. The mixture was cooled to 0°C, added dropwise during 30 min with 6.1 g methanesulfonyl chloride, stirred for 3 h, and then gradually heated to 20°C. TLC analysis showed that the reaction was complete. The mixture was added with 250 ml water and ice. A few minutes later, an abundant precipitate was formed, which was filtered, washed with water and dried at 50°C.
9.6 g of omega-mesyl acyclovir was obtained (yield 70.9%).

### Example 9 - Hydrated valacyclovir hydrocloride (I-A)

A 100 ml conical flask equipped with magnetic stirrer was fed with 10 ml DMF, 1 g omega-mesyl acyclovir prepared as described in Example 8 and 0.935 g L-valine sodium salt (V) prepared as described in Example 1.

The reaction mixture was stirred at 80°C for 3 h. After that time, TLC analysis showed the disappearance of the spot corresponding to omega-mesyl acyclovir.

The solution pH was brought to 2.0-2.5 by addition of 15% HCl at 20°C. TLC analysis showed the appearance of the spot corresponding to valacyclovir.

The mixture was concentrated under vacuum and the doughy residue was dissolved in 5 ml absolute ethanol and cooled to 0°C.

The crystalline precipitate was filtered, washed with little ethanol and dried.

0.72 g of valacyclovir hydrochloride having a water content of 6.5% was obtained (reaction yield 57% of theoretical value)

The I.R. spectrum of the product is shown in Fig. 1.

The ¹H NMR and mass spectral data are identical with those of the product obtained in Example 6.

### Example 10 - omega-Chloroacyclovir (IV)

A 100 ml conical flask was fed with 10 ml DMF, 1.0 g omega-mesyl acyclovir and 0.5 g lithium chloride. The mixture was heated to 60°C under a nitrogen stream. Three hours later, TLC analysis showed that the reaction was complete. The solution was cooled to 20°C, poured into 100 ml water, stirred for 10 min, filtered and washed with water. After drying at 50°C, 0.77 g of product was obtained (yield 96% of theoretical value).

The mass spectrum of the compound showed peaks at m/z: 243 (molecular ion), 208 (chlorine loss), 180 (CH₂CH₂Cl fragment loss), 164 (-0-CH₂CH₂-Cl fragment loss).

The ¹H NMR spectrum of the compound showed signals at δ 3.65-4.76 (4H,m); 4.40 (2H,s); 6.54 (2H,s) (disappears by deuteration); 7.82 (1H,s); 10.54 (1H,s) (disappears by deuteration).

### Example 11 - Anhydrous omega-valacyclovir hydrochloride (I-A)

A 25 ml conical flask was fed with 1.0 g omega-chloroacyclovir, 10 ml DMF, 1.0 g L-valine sodium salt (V). The mixture was heated to 80°C, added portionwise (one portion of approx. 100 mg every 30 min) with 0.5 g potassium iodide, allowed to stir at 95°C overnight, then cooled to 20°C and poured into 50 ml water at 15°C.

The precipitate was filtered and washed with 2x5 ml water.

The wet solid was suspended in 10 ml methanol, stirred at 15°C-20°C and brought to pH 2 by addition of 15% HCl. The resulting solution was concentrated to small volume and diluted with 10 ml isopropanol.

The solid obtained was filtered, recrystallized twice from methanol and dried.

1 g of valacyclovir hydrochloride having a water content of 0.4% was obtained (reaction yield 68% of theoretical value).

The I.R. spectrum of the product is shown in Fig. 2.

H¹ NMR and mass spectral data are identical with those of the product obtained in Example 6.

## Claims

1. Process of preparation of salified valacyclovir (I-A) 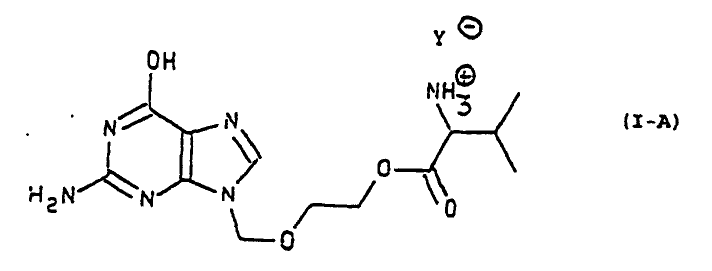 in which Y^{⊖} is selected f rom the group consisting of Cl^{⊖}, HSO₄^{⊖}, paratoluenesulfonate, methanesulfonate and trifluoromethane-sulfonate, or the corresponding free base (I) 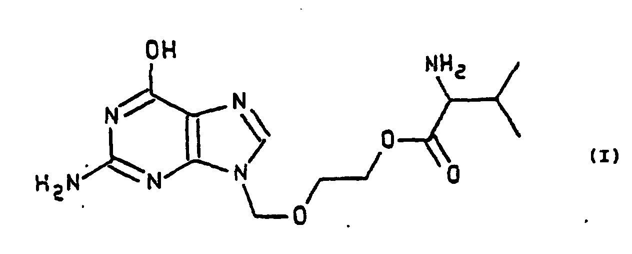 consisting of the following steps:
a) reacting acyclovir (II) 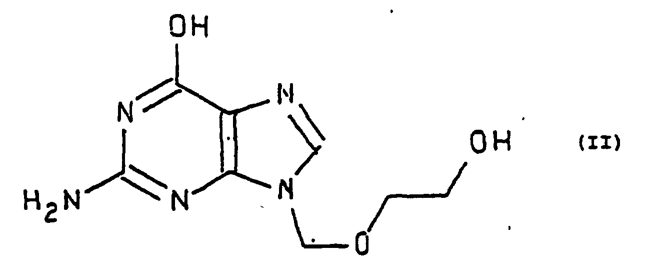 with a Z-L reactant selected from the group consisting of para-toluenesulfonyl chloride, methanesulfonyl chloride, trifluoromethanesulfonyl chloride, triflic anhydride, sulfuryl chloride, phosphorus tribromide and phosphorus trichloride, in the presence of a base and optionally a solvent, to give a compound of formula (IV) 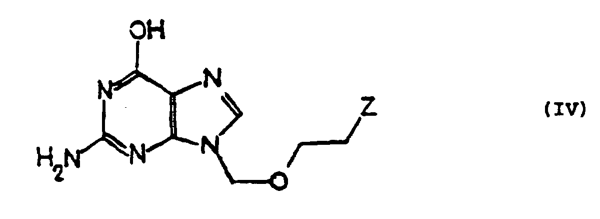 in which Z is selected from the group consisting of paratoluenesulfuryl, methanesulfuryl, trifluoromethanesulfuryl, Cl and Br;
b) reacting product (IV) coming from the step (a) with an alkaline salt of L-valine of formula (V) 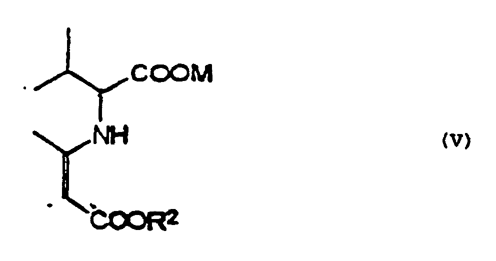 in which R² is a C₁-C₁₀ alkyl and M is an alkali metal,
in the presence of a high-boiling organic solvent selected from the group consisting of a dipolar aprotic solvent and an ether, at a temperature ranging from 20°C to 150°C, to give ester (VI) 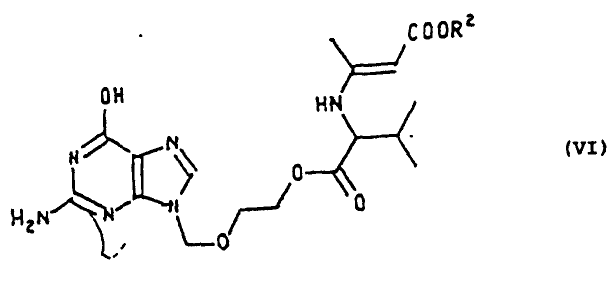 in which R² has the above meanings;
c) transforming product (VI) coming from the step (b) into the corresponding salified valacyclovir (I-A) by acid hydrolysis with a strong acid of mineral or organic type, at a temperature ranging from 0°C to 50°C, in water and/or in an organic solvent selected from the group consisting of the solvent used in step (b), an alcohol containing 1 to 4 carbon atoms, an ether, and a mixture of said alcohol and said ether;
d) optionally converting salified valacyclovir (I-A) coming from the step (c) into the corresponding free base (I) by conventional methods.

2. The process as claimed in claim 1, wherein the base used in step
(a) is selected from the group consisting of potassium carbonate, sodium methylate and pyridine.

3. The process as claimed in any preceding claim, wherein the solvent used in step (a) is selected from the group consisting of N,N-dimethylformamide, dimethylsulfoxide, pyridine and mixtures thereof.

4. The process as claimed in any of claims 1 and 2, wherein pyridine or a pyridine-dimethylformamide mixture is used in step (a) as a solvent or as a base.

5. The process as claimed in any of claims 1 to 4, wherein para-toluenesulfonyl chloride or methanesulfonyl chloride is used in step
(a) as a Z-L reactant.

6. The process as claimed in any of claims 1 to 5, wherein, when a dipolar aprotic solvent is used in step (b), said solvent is selected from the group consisting of N,N-dimethylformamide and dimethylsulfoxide.

7. The process as claimed in any of claims 1 to 5, wherein, when a high-boiling ether is used in step (b), said ether is selected from the group consisting of dioxane and diglyme.

8. The process as claimed in any of claims 1 to 7, wherein step (b) is carried out at a temperature ranging from 70°C to 90°C.

9. The process as claimed in any of claims 1 to 7, wherein, when the reactant used in step (b) is intermediate (IV), in which Z=Cl, potassium iodide, is further added.

10. The process as claimed in any of claims 1 to 9, wherein, when a mineral strong acid is used in step (c), said acid is selected from the group consisting of sulphuric acid and hydrochloric acid.

11. The process as claimed in any of claims 1 to 9, wherein, when an organic strong acid is used in step (c), said acid is selected from the group consisting of para-toluenesulfonic acid, methanesulfonic acid and trifluoromethanesulfonic acid.

12. The process as claimed in any of claims 1 to 11, wherein the aforesaid acid is added in quantities securing constant pH values of 2 to 3.

13. The process as claimed in any of claims 1 to 12, wherein, when an alcohol containing 1 to 4 carbon atoms is used as a solvent in step (c), said alcohol is methanol.

14. The process as claimed in any of claims 1 to 12, wherein, when an ether is used as a solvent in step (c), said ether is selected from the group consisting of dioxane and tetrahydrofuran.

15. The process as claimed in any of claims 1 to 12, wherein, when a mixture of an alcohol and of an ether is used in step (c), said mixture consists of methanol and tetrahydrofuran.

16. The process as claimed in any of claims 1 to 12, wherein step (c) is directly conducted on the reaction mixture obtained in step (b), without isolating intermediate (VI).

17. The process as claimed in any of claims 1 to 16, wherein step (d) includes the addition of an ammonium hydroxide aqueous solution to salified valacyclovir (I-A) solution to adjust the pH to a constant value ranging from 9 to 9.5.

18. Substituted omega-acyclovir derivative of formula (IV) 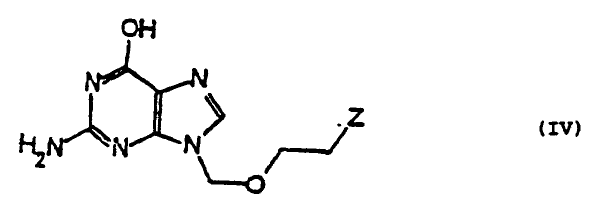 in which Z is selected from the group consisting of paratoluenesulfuryl, methanesulfuryl, trifluoromethanesulfuryl, Cl and Br.

19. Acyclovir ester of formula (VI) 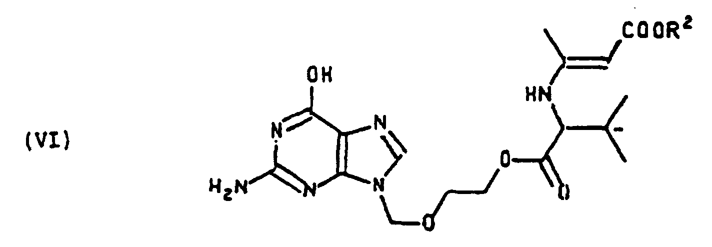 in which R² is C₁-C₁₀ alkyl.

20. An acyclovir ester as claimed in claim 19, wherein R² is selected from the group consisting of methyl, ethyl, n-propyl, isopropyl, n-butyl and isobutyl.

21. L-valine alkaline salt of formula (V) 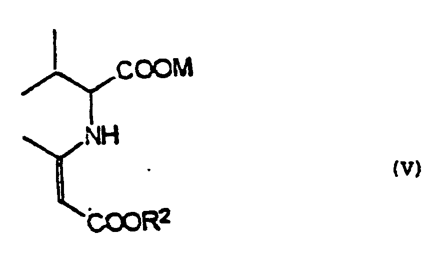 in which M is an alkali metal and R² is C₁-C₁₀ alkyl.

22. The L-valine alkaline salt as claimed in claim 21, wherein M is selected from the group consisting of sodium and potassium, and R² is selected from the group consisting of methyl, ethyl, n-propyl, isopropyl, n-butyl and isobutyl.

23. A process for preparing the L-valine alkaline salt as claimed in any of claims 21 and 22, comprising reacting L-valine in the presence of an alkaline hydroxide, with an alkyl acetoacetate of formula (VII) 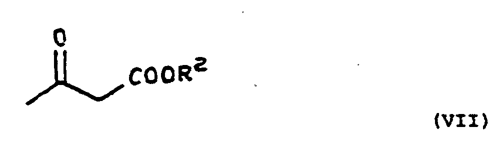 in which R² is C₁-C₁₀ alkyl, in a solvent selected from the group consisting of an alcohol containing 1 to 4 carbon atoms, a ketone and an aromatic hydrocarbon.

24. The process as claimed in claim 23, wherein, when an alcoholic solvent containing 1 to 4 carbon atoms is used, said solvent is methanol.

25. The process as claimed in claim 23, wherein, when a keto solvent is used, said solvent is selected from the group consisting of methylethylketone, diethylketone and methylisobutylketone.

26. The process as claimed in claim 23, wherein, when an aromatic hydrocarbon is used as a solvent, said solvent is selected from the group consisting of toluene and xylene.

## Patentansprüche

1. Verfahren zur Herstellung von Valaciclovir-Salz (I-A) 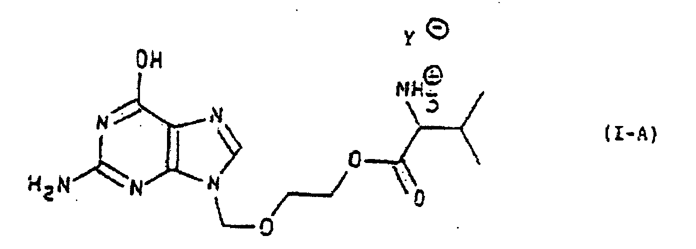 wobei Y⁻ ausgewählt wird aus der Gruppe bestehend aus Cl⁻, HSO4⁻, para-Toluolsulfonat, Methansulfonat und Trifluormethansulfonat, oder der korrespondierenden freien Base (I) 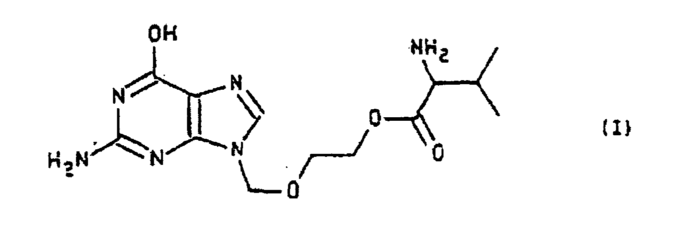 bestehend aus den folgenden Schritten:
a) Reagieren von Aciclovir (II) 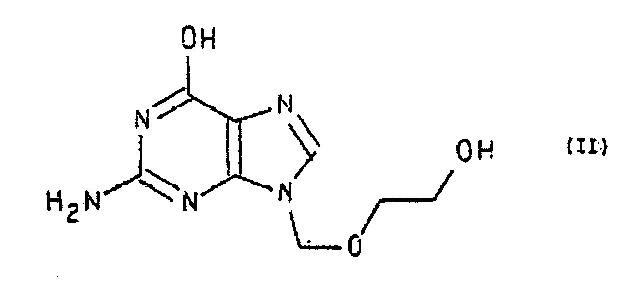 mit einem Z-L-Reaktanden ausgewählt aus der Gruppe bestehend aus para-Toluolsulfonylchlorid, Methansulfonylchlorid, Trifluoromethansulfonylchlorid, Trifluormethansäureanhydrid, Sulfurylchlorid, Phosphortribromid und Phosphortrichlorid, in Gegenwart einer Base und gegebenenfalls eines Lösungsmittels, um eine Verbindung mit der Formel (IV) zu erhalten, 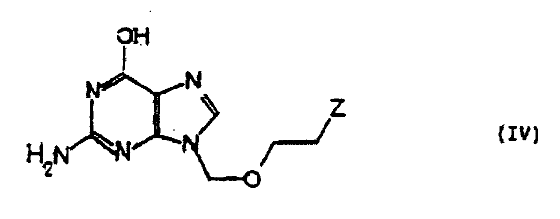 wobei Z ausgewählt wird aus der Gruppe bestehend aus para-Toluolsulfuryl-, Methansulfuryl-, Trifluormethansulfuryl-, Cl und Br;
b) Reagieren des Produkts (IV) aus Schritt a) mit einem Alkalisalz von L-Valin der Formel (V) 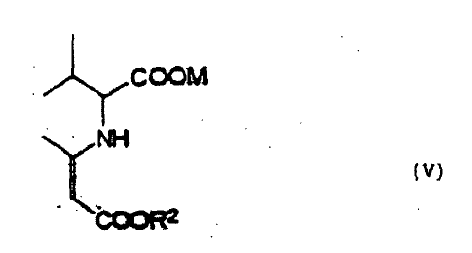 in welcher R² eine C₁-C₁₀-Alkylgruppe und M ein Alkalimetall ist, in Gegenwart eines hochsiedenden organischen Lösungsmittels ausgewählt aus der Gruppe bestehend aus einem dipolaren, aprotischen Lösungsmittel und einem Ether bei einer Temperatur zwischen 20 °C und 150 °C, um einen Ester (VI) zu erhalten, 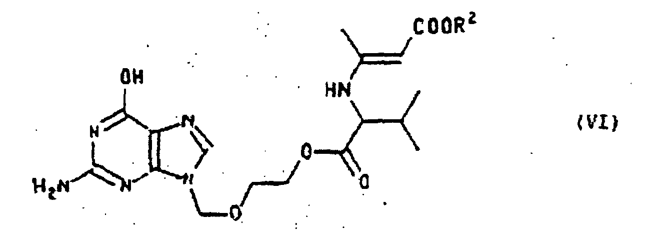 in welcher R² die oben genannten Bedeutungen hat;
c) Umwandlung des Produktes (VI) aus Schritt b) in das korrespondierende Valaciclovir-Salz (I-A) durch Säurehydrolyse mit einer starken anorganischen oder organischen Säure bei einer Temperatur zwischen 0 °C und 50 °C in Wasser und/oder in einem organischen Lösungsmittel ausgewählt aus der Gruppe bestehend aus den in Schritt b) verwendeten Lösungsmitteln oder Alkohol mit 1 bis 4 Kohlenstoffatomen, einem Ether und einer Mischung aus dem genannten Alkohol und dem genannten Ether;
d) gegebenenfalls Umwandlung des Valaciclovir-Salzes (I-A) aus Schritt c) in die korrespondierende freie Base (I) mittels herkömmlicher Methoden.

2. Verfahren gemäß Anspruch 1, wobei die in Schritt a) verwendete Base ausgewählt wird aus der Gruppe bestehend aus Kaliumcarbonat, Natriummethylat und Pyridin.

3. Verfahren gemäß jedem der vorhergehenden Ansprüche, wobei das in Schritt a) verwendete Lösungsmittel ausgewählt wird aus der Gruppe bestehend aus N,N-Dimethylformamid, Dimethylsulfoxid, Pyridin und Mischungen daraus.

4. Verfahren gemäß jedem der Ansprüche 1 und 2, wobei in Schritt a) Pyridin oder eine Pyridin-Dimethylformamid-Mischung als Lösungsmittel oder Base verwendet wird.

5. Verfahren gemäß jedem der Ansprüche 1 bis 4, wobei in Schritt a) para-Toluolsulfonylchlorid oder Methansulfonylchlorid als Z-L-Reaktand verwendet wird.

6. Verfahren gemäß jedem der Ansprüche 1 bis 5, wobei, wenn in Schritt b) ein dipolares aprotisches Lösungsmittel verwendet wird, dieses Lösungsmittel ausgewählt wird aus der Gruppe bestehend aus N,N-Dimethylformamid und Dimethylsulfoxid.

7. Verfahren gemäß jedem der Ansprüche 1 bis 5, wobei, wenn in Schritt b) ein hochsiedender Ether verwendet wird, dieser Ether ausgewählt wird aus der Gruppe bestehend aus Dioxan und Diglyme.

8. Verfahren gemäß jedem der Ansprüche 1 bis 7, wobei Schritt b) ausgeführt wird bei einer Temperatur zwischen 70 °C und 90 °C.

9. Verfahren gemäß jedem der Ansprüche 1 bis 7, wobei, wenn der in Schritt b) verwendete Reaktand das Intermediat (IV) mit Z = Cl ist, zusätzlich Kaliumiodid zugegeben wird.

10. Verfahren gemäß jedem der Ansprüche 1 bis 9, wobei, wenn in Schritt c) eine anorganische starke Säure verwendet wird, diese Säure ausgewählt wird aus der Gruppe bestehend aus Schwefelsäure und Salzsäure.

11. Verfahren gemäß jedem der Ansprüche 1 bis 9, wobei, wenn in Schritt c) eine organische starke Säure verwendet wird, diese Säure ausgewählt wird aus der Gruppe bestehend aus para-Toluolsulfonsäure, Methansulfonsäure und Trifluormethansulfonsäure.

12. Verfahren gemäß jedem der Ansprüche 1 bis 11, wobei die vorgenannte Säure in Mengen zugegeben wird, die einen konstanten pH-Wert von 2 bis 3 sichern.

13. Verfahren gemäß jedem der Ansprüche 1 bis 12, wobei, wenn in Schritt c) ein Alkohol mit 1 bis 4 Kohlenstoffatomen als Lösungsmittel verwendet wird, dieser Alkohol Methanol ist.

14. Verfahren gemäß jedem der Ansprüche 1 bis 12, wobei, wenn in Schritt c) ein Ether als Lösungsmittel verwendet wird, dieser Ether ausgewählt wird aus der Gruppe bestehend aus Dioxan und Tetrahydrofuran.

15. Verfahren gemäß jedem der Ansprüche 1 bis 12, wobei, wenn in Schritt c) eine Mischung aus einem Alkohol und einem Ether verwendet wird, diese Mischung aus Methanol und Tetrahydrofuran besteht.

16. Verfahren gemäß jedem der Ansprüche 1 bis 12, wobei Schritt c) direkt auf die in Schritt b) erhaltene Reaktionsmischung angewandt wird, ohne das Intermediat (VI) zu isolieren.

17. Verfahren gemäß jedem der Ansprüche 1 bis 16, wobei Schritt d) die Zugabe einer wässrigen Lösung von Ammoniumhydroxid zur Valaciclovir-Salz-Lösung (I-A) einschließt, um den pH-Wert auf einen konstanten Wert von 9 bis 9,5 einzustellen.

18. Substituiertes Omega-Aciclovir der Formel (IV) 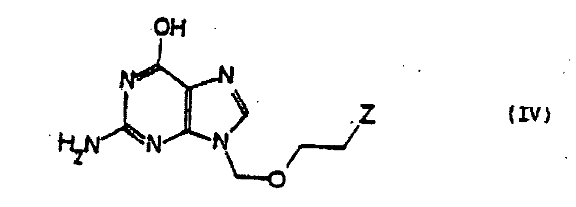 wobei Z ausgewählt wird aus der Gruppe bestehend aus para-Toluolsulfuryl-, Methansulfuryl-, Trifluormethansulfuryl-, Cl und Br.

19. Ein Aciclovir-Ester mit der Formel (VI) 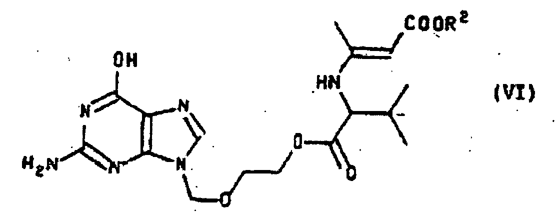 wobei R² eine C₁-C₁₀-Alkylgruppe ist.

20. Ein Aciclovir-Ester gemäß Anspruch 19, wobei R² ausgewählt wird aus der Gruppe bestehend aus Methyl-, Ethyl-, n-Propyl-, Isopropyl-, n-Butyl- und Isobutyl-.

21. Ein L-Valinalkalimetallsalz mit der Formel (V) 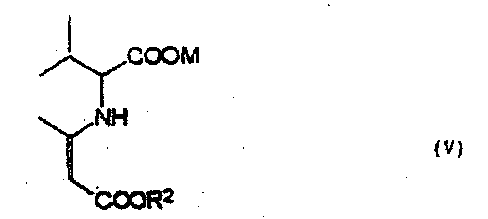 wobei M ein Alkalimetall und R² eine C₁-C₁₀-Alkylgruppe ist.

22. Das L-Valinalkalimetallsalz gemäß Anspruch 21, wobei M ausgewählt wird aus der Gruppe bestehend aus Natrium und Kalium und R² ausgewählt wird aus der Gruppe bestehend aus Methyl-, Ethyl-, n-Propyl-, Isopropyl-, n-Butyl- und Isobutyl-.

23. Ein Verfahren zur Herstellung des L-Valinalkalimetallsalzes gemäß jedem der Ansprüche 21 und 22, umfassend die Reaktion von L-Valin in Gegenwart eines Alkalimetallhydroxids mit einem Alkylacetoacetat mit der Formel (VII) 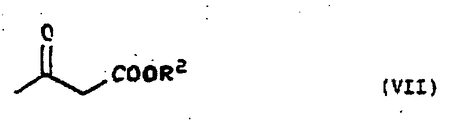 wobei R² eine C₁-C₁₀-Alkylgruppe ist, in einem Lösungsmittel ausgewählt aus der Gruppe bestehend aus einem Alkohol mit 1 bis 4 Kohlenstoffatomen, einem Keton und einem aromatischen Kohlenwasserstoff.

24. Ein Verfahren gemäß Anspruch 23, wobei, wenn ein alkoholisches Lösungsmittel mit 1 bis 4 Kohlenstoffatomen verwendet wird, dieses Lösungsmittel Methanol ist.

25. Verfahren gemäß Anspruch 23, wobei, wenn ein Keton als Lösungsmittel verwendet wird, dieses Lösungsmittel ausgewählt wird aus der Gruppe bestehend aus Methylethylketon, Diethylketon und Methylisobutylketon.

26. Verfahren gemäß Anspruch 23, wobei, wenn ein aromatischer Kohlenwasserstoff als Lösungsmittel verwendet wird, dieses Lösungsmittel ausgewählt wird aus der Gruppe bestehend aus Toluol und Xylol.

## Revendications

1. Procédé de préparation de valacyclovir (I-A) salifié 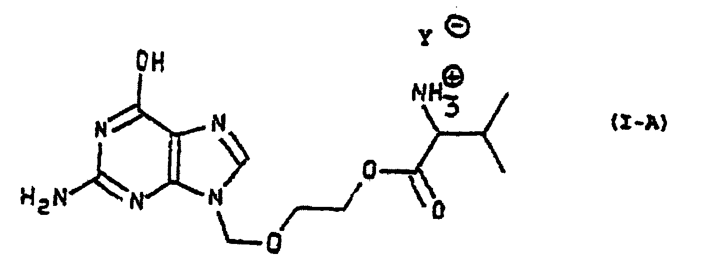 où Y⁻ est sélectionné dans le groupe consistant en Cl⁻, HSO₄⁻ paratoluènesulfonate, méthanesulfonate et trifluoromethane-sulfonate, ou la base libre correspondante (I) 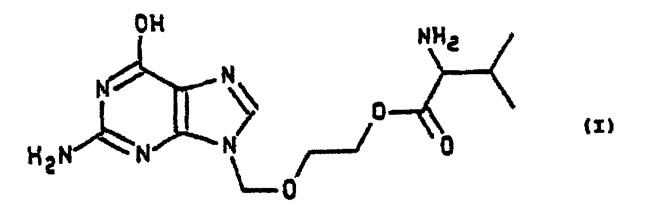 consistant en les étapes suivantes :
a) réaction de l'acyclovir (II) 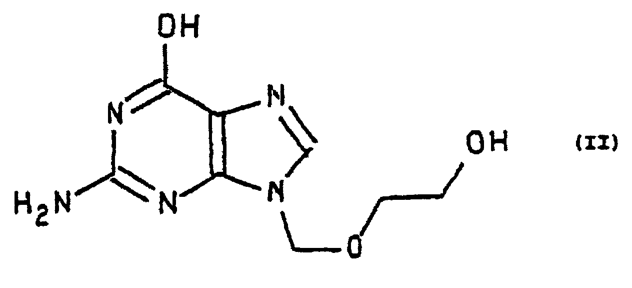 avec un réactant Z-L sélectionné dans le groupe consistant en chlorure de paratoluènesulfonyle, chlorure de méthanesulfonyle, chlorure de trifluorométhanesulfonyle, anhydride triflique, chlorure de sulfuryle, tribromure de phosphore et trichlorure de phosphore, en présence d'une base et facultativement d'un solvant pour donner un composé de la formule (IV) 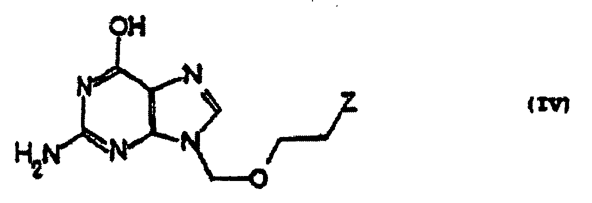 où Z est sélectionné dans le groupe consistant en paratoluènesulfuryle, méthanesulfuryle, trifluorométhanesulfuryle, Cl et Br ;
b) réaction du produit (IV) provenant de l'étape (a) avec un sel alcalin de L-valine de la formule (V) 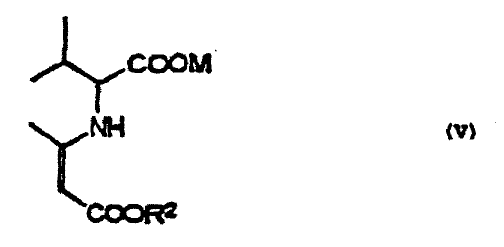 dans laquelle R² est un alkyle C₁-C₁₀ et M est un métal alcalin,
en présence d'un solvant organique de fort point d'ébullition sélectionné dans le groupe consistant en un solvant aprotique dipolaire et un éther, à une température allant de 20°C à 150°C, pour donner l'ester (VI) 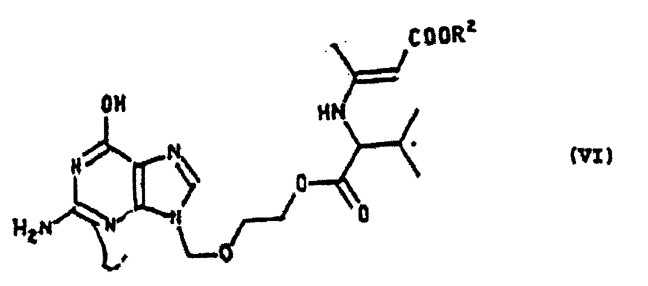 où R² a les significations ci-dessus ;
c) la transformation du produit (VI) provenant de l'étape (b) en valacyclovir (I-A) salifié correspondant par hydrolyse acide avec un acide fort de type minéral ou organique, à une température allant de 0°C à 50°C, dans l'eau et/ou dans un solvant organique sélectionné dans le groupe consistant en le solvant utilisé à l'étape (b), un alcool contenant 1 à 4 atomes de carbone, un éther et un mélange dudit alcool et dudit éther ;
d) facultativement, la conversion du valacyclovir (I-A) salifié provenant de l'étape (c) en base libre (I) correspondant par des méthodes conventionnelles.

2. Procédé selon la revendication 1, où la base utilisée à l'étape (a) est sélectionnée dans le groupe consistant en carbonate de potassium, méthylate de sodium et pyridine.

3. Procédé selon toute revendication précédente, où le solvant utilisé à l'étape (a) est sélectionné dans le groupe consistant en N,N-diméthylformamide, diméthylsulfoxyde, pyridine et leurs mélanges.

4. Procédé selon l'une quelconque des revendications 1 à 3, où on utilise de la pyridine ou un mélange pyridine-dimethylformamide à l'étape (a), en tant que solvant ou en tant que base.

5. Procédé selon l'une quelconque des revendications 1 à 4, où du chlorure de paratoluènesulfonyle ou du chlorure de méthanesulfonyle est utilisé à l'étape (a) en tant que réactant Z-L.

6. Procédé selon l'une quelconque des revendications 1 à 5 où, quand un solvant aprotique dipolaire est utilisé à l'étape (b), ledit solvant est sélectionné dans le groupe consistant en N,N-diméthylformamide et diméthylsulfoxyde.

7. Procédé selon l'une quelconque des revendications 1 à 5 où, quand un éther de fort point d'ébullition est utilisé à l'étape (b), ledit éther est sélectionné dans le groupe consistant en dioxane et diglyme.

8. Procédé selon l'une quelconque des revendications 1 à 7 où l'étape (b) est effectuée à une température allant de 70°C à 90°C.

9. Procédé selon l'une quelconque des revendications 1 à 7 où, quand le réactant utilisé à l'étape (b) est l'intermédiaire (IV), où Z = Cl, de l'iodure de potassium est de plus ajouté.

10. Procédé selon l'une quelconque des revendications 1 à 9 où, quand un acide minéral fort est utilisé à l'étape (c), ledit acide est sélectionné dans le groupe consistant en l'acide sulfurique et l'acide chlorhydrique.

11. Procédé selon l'une quelconque des revendication 1 à 9 où, quand un acide organique fort est utilisé à l'étape (c), ledit acide est sélectionné dans le groupe consistant en acide paratoluènesulfonique, acide méthanesulfonique et acide trifluorométhanesulfonique.

12. Procédé selon l'une quelconque des revendication 1 à 11, où l'acide ci-dessus est ajouté en quantités assurant des valeurs constantes de pH de 2 à 3.

13. Procédé selon l'une quelconque des revendications 1 à 12 où, quand un alcool contenant 1 à 4 atomes de carbone est utilisé comme solvant à l'étape (c), ledit alcool est du méthanol.

14. Procédé selon l'une quelconque des revendications 1 à 12, où, quand un éther est utilisé comme solvant à l'étape (c), ledit éther est sélectionné dans le groupe consistant en dioxane et tétrahydrofuranne.

15. Procédé selon l'une quelconque des revendications 1 à 12 où, quand un mélange d'un alcool et d'un éther est utilisé à l'étape (c ), ledit mélange consiste en méthanol et tetrahydrofuranne.

16. Procédé selon une quelconque des revendications 1 à 12, où l'étape (c) est directement entreprise sur le mélange réactionnel obtenu à l'étape (b), sans isoler l'intermédiaire (VI).

17. Procédé selon l'une quelconque des revendications 1 à 16, où l'étape (d) comprend l'addition d'une solution aqueuse d'hydroxyde d'ammonium à la solution de valacyclovir
(I-A) salifié pour ajuster le pH à une valeur constante allant de 9 à 9,5.

18. Dérivé substitué d'oméga-acyclovir de la formule (IV) 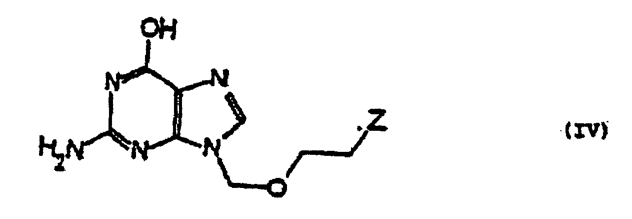 dans laquelle Z est sélectionné dans le groupe consistant en paratoluènesulfuryle, méthanesulfuryle, trifluorométhanesulfuryle, Cl et Br.

19. Ester d'acyclovir de la formule (VI) 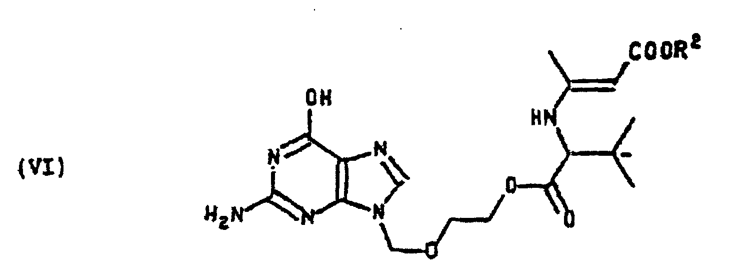 dans laquelle R² est alkyle C₁-C₁₀

20. Ester d'acyclovir selon la revendication 19, où R² est sélectionné dans le groupe consistant en méthyle, éthyle, n-propyle, isopropyle, n-butyle et isobutyle.

21. Sel alcalin de L-valine de la formule (V) 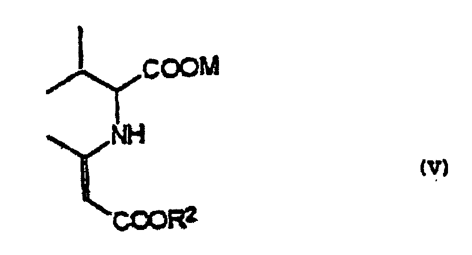 dans lequel M est un métal alcalin et R² est alkyle C₁-C₁₀.

22. Sel alcalin de L-valine selon la revendication 21, où M est sélectionné dans le groupe consistant en sodium et potassium et R² est sélectionné dans le groupe consistant en méthyle, éthyle, n-propyle, isopropyle, n-butyle et isobutyle.

23. Procédé de préparation du sel alcalin de L-valine selon l'une quelconque des revendications 21 et 22, comprenant la réaction de L-valine en présence d'un hydroxyde alcalin avec un acétoacétate alcalin de la formule (VII) 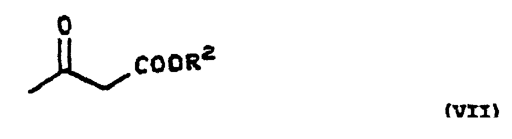 où R² est alkyle C₁-C₁₀, dans un solvant sélectionné dans le groupe consistant en un alcool contenant 1 à 4 atomes de carbone, une cétone et un hydrocarbure aromatique.

24. Procédé selon la revendication 23, où, quand un solvant alcoolisé contenant 1 à 4 atomes de carbone est utilisé, ledit solvant est du méthanol.

25. Procédé selon la revendication 23, où, quand un céto-solvant est utilisé, ledit solvant est sélectionné dans le groupe consistant en méthyléthylcétone, diéthylcétone et méthylisobutylcétone.

26. Procédé selon la revendication 23 où, quand un hydrocarbure aromatique est utilisé comme solvant, ledit solvant est sélectionné dans le groupe consistant en toluène et xylène.
